# EUROPEAN PATENT SPECIFICATION

(11) **EP 0 591 191 B1**
(45) Date of publication and mention of the grant of the patent: **08.09.1999**
(21) Application number: 91913930.3
(22) Date of filing: 23.07.1991
(51) Int. Cl.: A61F 9/00

(54) **APPARATUS FOR PERFORMING CORNEAL RESHAPING TO CORRECT OCULAR REFRACTIVE ERRORS**
VORRICHTUNG FÜR DIE HORNHAUTUMFORMUNG ZUM KORRIGIEREN VON BRECHUNGSFEHLERN DES AUGES
APPAREIL DE MODIFICATION DE LA FORME DE LA CORNEE POUR CORRIGER LES VICES DE REFRACTION DANS L'OEIL

(30) Priority: 23.07.1990 US 556886; 11.10.1990 US 596060; 20.05.1991 US 702960
(43) Date of publication of application: 13.04.1994
(73) Proprietor: LASER BIOTECH, INC., Fremont, California 94538 (US)
(72) Inventor: BERRY, Michael, J., The Woodlands, TX 77381 (US)
(74) Representative: Hitchcock, Esmond Antony
(86) International application number: US9105187
(87) International publication number: WO9201430

(56) References cited:
- EP-A- 0 190 829
- EP-A- 0 195 375
- DD-A- 263 447
- US-A- 3 327 712
- US-A- 4 840 175
- US-A- 4 887 592

## Description

This invention relates to a noninvasive ophthalmological apparatus for reshaping the anterior surface of the cornea in order to achieve emmetropia (i.e., normal vision characterized by the absence of ocular refractive error; the emmetropic eye focuses parallel rays of light onto the retina to produce a clear image). It further relates to the use of light to induce thermal changes in the stromal collagen of the cornea to shrink the stromal collagen to produce the desired reshaping of the cornea.

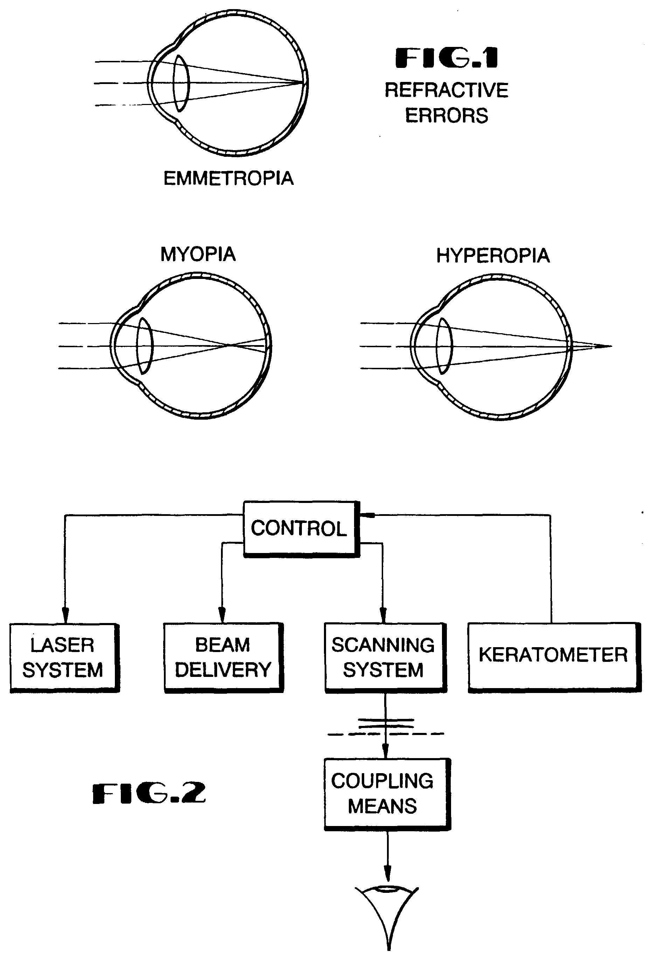

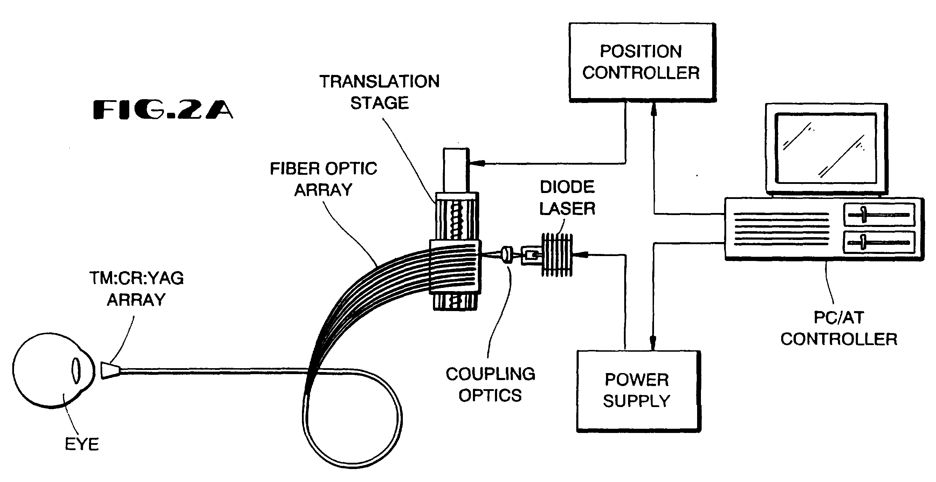

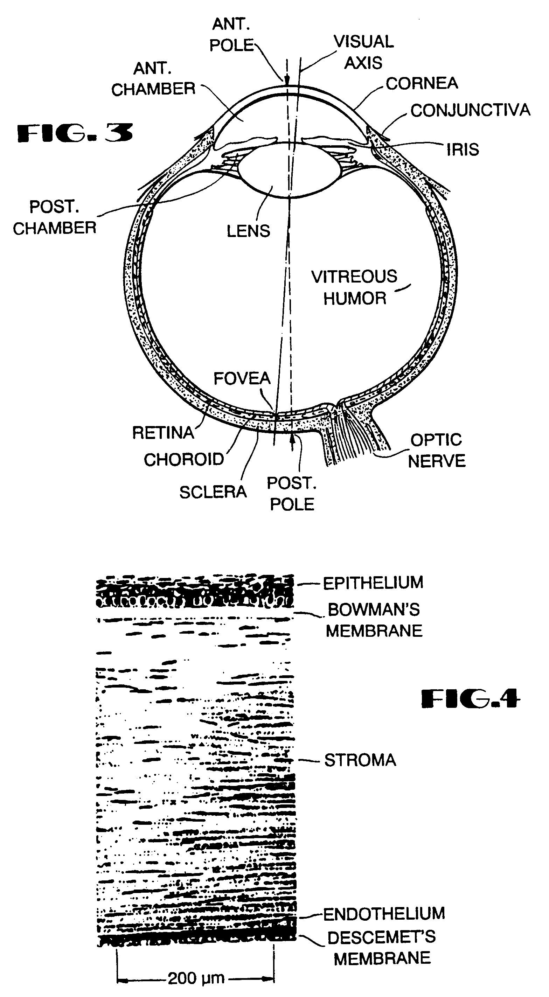

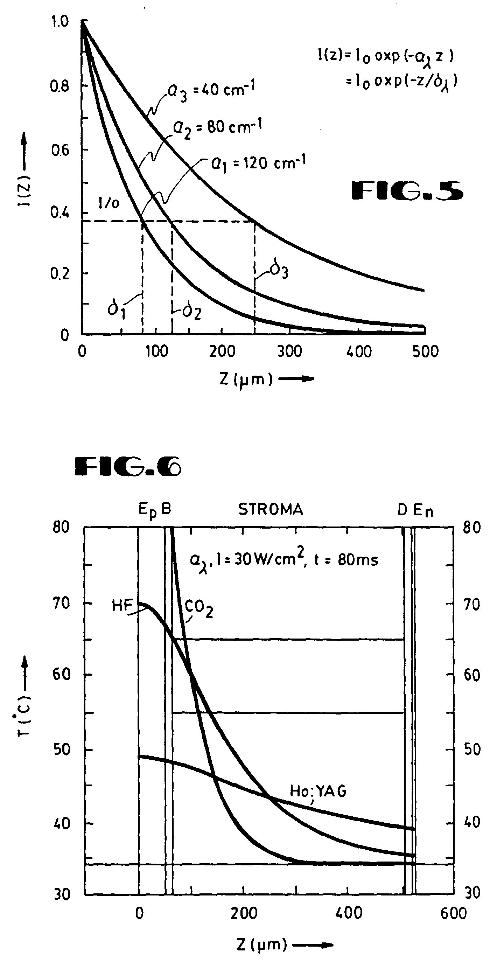

Today there are over 100 million people in the United States alone who wear eyeglasses or contact lenses to correct ocular refractive errors. The most common ocular refractive errors include myopia (nearsightedness), hyperopia (farsightedness), and astigmatism (See Figure 1). In myopia, the refractive power of the eye is excessive meaning that parallel rays of light are focused in front of the retina producing a blurred image. Myopic vision can be corrected by adding a spherical concave lens of the correct spherical curvature in front of the eye or by flattening the cornea axisymmetrically around the visual axis to reduce its refractive power.

In hyperopia (also termed hypermetropia), the refractive power of the eye is deficient meaning that parallel rays of light are focused behind the retina producing a blurred image. Hyperopic vision can be corrected by adding a spherical convex lens of the correct spherical curvature in front of the eye or by steepening the cornea axisymmetrically around the visual axis to increase its refractive power.

In astigmatism, the refractive power of the eye is unequal in all meridians meaning that parallel rays of light are focused differently along different meridians producing a blurred image. Astigmatic vision can be corrected by adding a non-spherical lens of the correct cylindrical curvatures along various meridians in front of the eye or by flattening and/or steepening the cornea with the correct cylindrical curvatures to compensate for refractive errors along various meridians.

Current widely used devices or methods for correcting ocular refractive errors include eyeglasses, contact lenses and refractive surgery such as radial keratotomy. Eyeglasses and contact lenses may be inconvenient, difficult to wear or impediments in daily activities.

Refractive surgery procedures offer an alternative to eyeglasses and contact lenses but these procedures may be difficult to control in order to achieve accurate refractive corrections. Radial keratotomy is a refractive surgical procedure designed to correct myopia. This technique involves making a series of deep, radial incisions in the cornea with a pattern that resembles the spokes of a bicycle wheel. The incisions themselves do not cross the center of the cornea, the optical zone. The series of symmetrical cuts flatten the cornea.

Significant percentages of patients who have been subjected to radial keratotomy experience overcorrection, undercorrection or induced astigmatism. Radial keratotomy patients may also suffer from side effects and postoperative complications such as fluctuating refraction, glare, reduced night vision, photophobia, endothelial cell loss, and corneal infection. Another postoperative complication of radial keratotomy is permanent weakening of the cornea due to the fact that the technique requires deep incisions that heal quite slowly. Trauma to the eyes may result in the rupture of the incisions leading to catastrophic loss of the cornea in some cases.

Similarly US 4,840,175 discloses a method for magnifying the curvature of a live cornea via use of a excimer laser. The live cornea has a thin layer removed therefrom having an exposed internal surface thereon. Then, either the surface or thin layer is exposed to the laser beam along a predetermined pattern to ablate desired portions. The thin layer is then replaced onto the surface. Ablating a central area makes the cornea less curved, while ablating an annular area spaced from the centre of the surface or layer makes the cornea more curved. The desired predetermined pattern is formed by use of a variable diaphragm, a rotating orifice of variable size, a movable mirror or a movable fibre optic cable through which the laser beam is directed towards the exposed internal surface or removed thin layer.

Another method of refractive surgery is laser keratomileusis (i.e., carving the cornea by application of laser energy) also termed laser refractive keratectomy or photorefractive keratectomy. This method of refractive surgery is currently being used in clinical trials in man to correct refractive errors. This technique employs the use of a laser that emits ultraviolet light, typically an argon fluoride excimer laser that operates at a wavelength of 193 nanometers. The laser light causes a breakdown of intramolecular bonds resulting in ablation of tissue by photodecomposition. The shape of the cornea is changed by selectively ablating material in the cornea thus "carving" the anterior corneal surface into a new shape. U.S. Patent No. 4,665,913 discloses one technique of photorefractive keratectomy.

For purposes of this application the "anterior" portion of the corneal stroma should be understood to refer to the first one third of the total depth of the corneal stroma. The stroma is typically 450 microns in total depth and therefore the anterior portion of the stroma should be understood to refer to the anterior-most 150 microns. Preferably the collagen rearrangement resulting from practice of this invention takes place in the anterior-most 100 microns of the stromal collagen and most preferably at a depth of 33-125 microns.

As is the case for other forms of refractive surgery, photorefractive keratectomy may lead to inadequate refractive corrections and to undesirable side effects. Particularly troublesome is the postoperative complication associated with corneal wound repair, a process that tends to "fill in" the ablated cornea volume with a combination of epithelial and stromal tissues. This process in the human cornea is sometimes referred to as a "wound-healing response". There are also concerns about the potential phototoxic effect of ultraviolet light generated by corneal tissue fluorescence and the potential toxic effect of molecular ablation products present in the photoablation plume.

Another method of refractive surgery is intrastromal photorefractive keratoplasty. In this technique a laser beam is focussed inside the corneal stromal tissue to modify tissue either by photoablation or by a change in the tissue's viscoelastic properties. U.S. Patent No. 4,907,586 discloses one such method. The wavelengths of the laser beams to be used are specified to be 526 nanometers, 1.053 microns, or 2.94 microns. Some of these wavelengths (526 nanometers and 1.053 microns) are transmitted, at least in part, through the cornea possibly causing damage to the retina. If laser induced optical breakdown (i.e., laser induced plasma formation) is used to increase the absorption of these wavelengths, the hot plasma will reradiate light with a broad wavelength distribution that includes phototoxic light in the ultraviolet spectral region. The final wavelength (2.94 microns) specified in U.S. Patent No. 4,907,586 is absorbed completely in the anterior portion (particularly, the epithelium) of the cornea [G.L. Valderrama, et al., *SPIE Proceedings*, Vol. 1064, 135-145 (1989)] so that it cannot produce intrastromal tissue modification. The alleged intrastromal photorefractive keratoplasty method is unworkable at some wavelengths and undesirable at other wavelengths because there may be severe damage caused to ocular structures.

Thermokeratoplasty is another method that has been used to reshape the cornea. This is done by the application of heat to the cornea. Corneal stromal collagen shrinks when heated to a temperature of 55° to 58°C, without the destruction of the tissue. If the pattern of shrinkage is properly selected the resulting change in the stress field and mechanical properties caused by the shrunken collagen fibers can be used to reshape the cornea.

The original thermokeratoplasty technique used was the application of a heated probe to the cornea leading to conductive heating of the stroma. However, the direct application of a heated probe is uncontrolled and unavoidably destructive. This technique caused thermal destruction of the epithelium as well as Bowman's membrane, the important tissue layer immediately underlying the epithelium. Some patients treated with this technique also showed damage to the deeper corneal stroma and endothelium. Additionally, this technique often involved inadequate refractive correction. Others have attempted the correction of hyperopia by using heated needles to burn a series of craters into the cornea. Recently the hot needles have been replaced with a laser in an effort to produce a more controlled thermal deposition. Severe damage to the corneal tissue still occurs and makes these procedures too undesirable for most ophthalmologists to recommend to their patients.

Another method of thermokeratoplasty involves microwave heating of the corneal stromal collagen. Microwave energy can be deposited deeply within the corneal stroma. U.S. Patent No. 4,881,543 discloses one method and apparatus for heating the central stroma of the cornea with microwave electromagnetic energy to the shrinking temperature of the collagen while circulating a cool fluid over the anterior surface of the cornea. However, microwave thermokeratoplasty procedures do not provide the spatial and temporal resolution and control required to perform accurate corneal reshaping without excessive thermal damage to corneal structures.

Recent thermokeratoplasty techniques are disclosed in the following references: U.S. Patent Number 4,976,709 issued on December 11, 1990 to Bruce J. Sand entitled "Method For Collagen Treatment"; and Patent Cooperation Treaty Publication Number WO 90/12618 by Kenneth Spears et al. published on November 1, 1990 entitled "Laser Thermal Keratoplasty System". Both of these references disclose and require laser wavelengths and absorption coefficients that result in collagen shrinkage throughout the entire depth of the corneal tissue.

These recent thermokeratoplasty references teach thermokeratoplasty using lasers operating in a frequency range from 1.8 to 2.55 microns with an absorption coefficient range of 15-120 cm⁻¹. These devices also use an air coolant applied to the anterior cornea to prevent heat damage to the epithelium and Bowman's membrane.

However, at these short wavelengths long irradiation times are required and collagen shrinkage occurs deeper in the stroma. These references do not stress providing adequate cooling for the anterior of the cornea if longer wavelengths are used and do not mask out stray light from areas which do not require treatment to prevent accidental damage to these layers if light should irradiate them. Accidental light irradiation would be even more catastrophic if longer wavelength light were used.

The present invention is directed at means by which the cornea can be reshaped in a safe, effective and dependable manner. Particularly, the invention seeks to enable the safe use of light with sufficiently high spatial/temporal resolution and control to reshape the cornea by controlled "heat treatment" of the stromal collagen. Suitable apparatus comprises a source of optical radiation of a wavelength suitable for absorption by tissue containing collagen tissue in the cornea; means for controlling the radiation source; and means for directing radiation from the source to the tissue. According to the invention the directing means in such apparatus comprises a plurality of optical fibres for receiving radiation from the source at first ends thereof and transmitting such radiation for delivery from second ends thereof, and a coupler for coupling radiation onto the outside face of a cornea is disposed adjacent the second ends of the optical fibres, the second ends thereof being positioned such that radiation spots can be delivered through the coupler to a cornea. Means are also provided for selectively locating such delivered spots of radiation in a pattern to the cornea, with the locating means and the controlling means being operable to modify the shape of a cornea by selective shrinkage therein of tissue containing collagen. Conveniently, the coupler may provide a mould for the curvature of the cornea.

In typical embodiments of the invention means are provided for illuminating the first ends of selected ones of the optical fibres with radiation from the source. This illuminating means include means for scanning a beam of said optical radiation among the first ends of selected ones of the optical fibres. In all embodiments, the second ends of the optical fibres may be positioned to deliver through the coupler to the cornea a pattern of radiation spots spaced from one another.

The invention will be further explained in the following description, given by way of example only, in which embodiments of the invention, and their use in various ocular treatments are discussed. Reference will be made to the accompanying drawings, wherein:
Figure 1 is a schematic representation of an emmetropic eye (top) and of eyes having common refractive errors (bottom) including a myopic eye (bottom left) and a hyperopic eye (bottom right). Astigmatism is a refractive error involving combinations of emmetropia, myopia, and/or hyperopia along different ocular meridians.
Figure 2 is a schematic diagram of the essential components of one embodiment of the apparatus used to perform corneal reshaping to correct ocular refractive errors.
Figure 2A is a schematic diagram of the essential components of the preferred embodiment of the apparatus used to perform corneal reshaping to correct ocular refractive errors.
Figure 3 is a schematic cross section of the human eye showing several ocular structures.
Figure 4 is a schematic representation of corneal microstructure showing important corneal structures.
Figure 5 shows the distribution of light irradiance I as a function of penetration distance z within corneal tissue for three values of absorption coefficients α at wavelengths λ = 1, 2 and 3. The initial irradiance I₀ decreases exponentially to 37% (1/e) of its value at a penetration depth δ that depends on the incident wavelength of the light. The penetration depth δ is the inverse of the absorption coefficient α indicated in the figure. Hence, for an absorption coefficient α of 80 cm⁻¹, the characteristic penetration depth δ is 125 µm.
Figure 6 shows the results of one-dimensional (1D) thermal modelling calculations of temperature distributions as a function of depth of penetration into corneal tissue. Typical depths of microstructural layers of the cornea are indicated for the epithelium (Ep), the Bowman's layer (B), the stroma, Descemet's membrane (D), and the endothelium (En). The calculations use estimated thermal properties (thermal conductivity, thermal diffusivity, and heat capacity) for human corneas together with optical absorption coefficients a pertinent to three laser wavelengths produced by three different laser sources (CO₂, HF, and Ho:YAG). All three calculations are for absorbed irradiances of 30 W/cm² and exposure durations of 80 ms. The desired temperature range (approximately 55°C to 65°C) for collagen shrinkage without gross thermal damage is shown within the corneal stroma.
Figure 7 shows thermal modelling calculations similar to those shown in Figure 6 except that different temperature distributions are plotted as a function of time (at fixed exposure durations of 1 ms through 10 ms) for application of a hydrogen fluoride chemical laser source (at a predetermined wavelength of approximately λ = 2.61 µm) at a fixed irradiance of 400 W/cm². Note that the corneal endothelium remains cool in all cases.

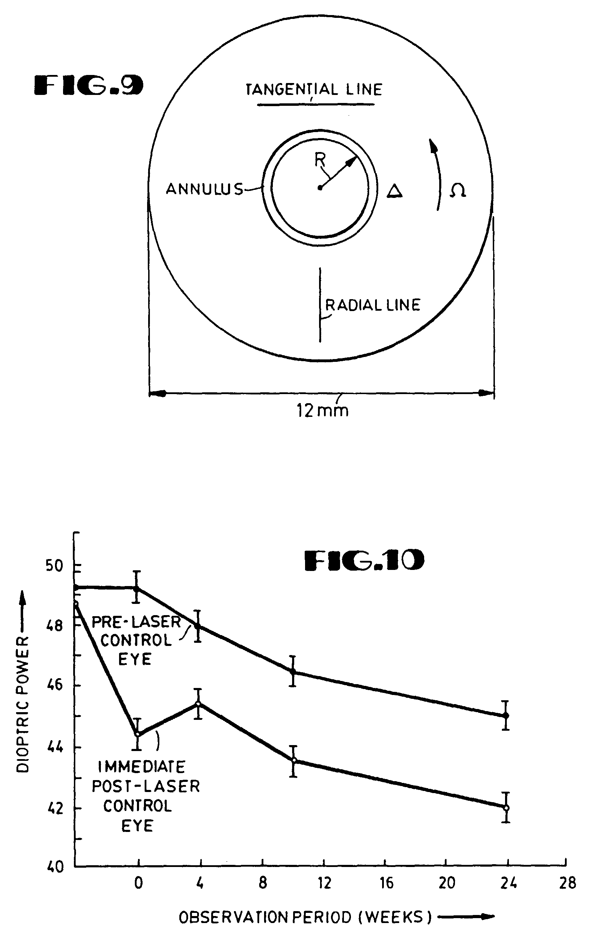

Figure 8 shows thermal modelling calculations similar to those shown in Figure 6 except that the temperature distribution that peaks on the anterior surface (z = 0) of the cornea is for application of a hydrogen fluoride (HF) chemical laser source (at a predetermined wavelength of approximately λ = 2.61 µm) at a fixed irradiance of 30 W/cm² and a fixed time of 80 ms. The temperature distribution that peaks within the anterior portion of the stroma is calculated for the same laser wavelength but includes the effects of a heat sink/coupler in thermal contact with the anterior surface of the cornea. In this case, the HF chemical laser source is applied for a fixed irradiance of 100 W/cm² and a fixed time of 100 ms.
Figure 9 is a schematic representation of different patterns of treatment on the anterior surface of the cornea. The annulus has a radius R and a width Δ and is drawn using a laser spot that is slewed at an angular velocity Ω.
Figure 10 shows experimental corneal curvature (dioptric, or refractive, power) measurements for both untreated (control) and treated eyes of the example rabbit (Ignatieff) at different observation times.
Figure 11 is a schematic representation of the fiber optic array used in the apparatus of the preferred embodiment of the invention.
Figure 12 is a schematic representation of one strand of optical fiber used in the fiber optic array used in the apparatus of the preferred embodiment of the invention.

The techniques described herein include methods for reshaping the cornea to correct ocular refractive errors such as myopia, hyperopia and the delivery of the dose of photothermal radiation to the corneal tissue region can be controlled to that required to cause collagen shrinkage without photodecomposition and to produce accurate control of the corneal reshaping process. This process is known as photothermokeratoplasty. Light source parameters can be provided to assure safe and effective corneal reshaping. One embodiment of this invention provides for a heat sink at the anterior surface of the cornea to prevent damage to the epithelium and Bowman's membrane. Another embodiment provides for a solid heat sink to act as a template for the predetermined physical changes to the curvature of the corneal tissue. Still another embodiment provides a fibre optic array to control the light energy applied to the cornea.

This invention provides for the noninvasive reshaping of the corneal tissue by exposing the cornea to a functionally effective amount of light energy in a spatially localized pattern. The light energy is typically applied at one or more predetermined wavelengths between 1.87 - 2.08 or 2.55 - 2.7 microns, irradiance levels and time durations to thermally induce predetermined, physical changes to the curvature of the cornea. These changes can be induced without also inducing a wound-healing response in the stroma sufficient to significantly change the corneal curvature from its predetermined value and without damaging the viability of the corneal endothelium or the anterior surface of the corneal tissue. Provision can be made for the required physical changes to be induced by more than one exposure to a functionally effective amount of light energy. Depending on the severity of the treatment, it is possible to avoid exposure of the central optical zone during treatment. However, the decision as to whether or not to include the central optical zone in the spatial pattern of treatment is within the discretion of the patient.

In the use and practice of the invention one or more predetermined wavelengths, irradiance levels and time durations can be functionally effective to produce a predetermined change in only the anterior portion of the stroma. The preferred depth of penetration of light into the cornea is about 33 microns to about 125 microns. The preferred irradiance level of light energy is at a level where the absorption is substantially linear, and most preferably where the absorption coefficient is 30cm⁻¹ to 100 cm⁻¹ at the 1.87 - 2.08 micron wavelength range or 100 cm⁻¹ to 300 cm⁻¹ at the 2.55 - 2.7 micron wavelength range. The preferred time or duration of exposure is less than about one second in each portion of the pattern of application. The preferred light source is an erbium light source with the preferred wavelengths generated from the light source being about 2.55 to 2.7 microns. The light source may be either a coherent light source or a non-coherent light source. The most preferred embodiment is a non-coherent erbium light source. Other preferred light sources include holmium, thulium, and uranium based light sources, both coherent and non-coherent, and combinations thereof.

Experimentation has shown that it is essential that the stromal collagen temperature be preferably heated to about 55°C to 65°C during the thermal modification portion of the method although treatments in a temperature range of 55°C to 75°C have been functionally satisfactory. Below about 55°C there is no apparent physical effect and above 75°C there is permanent damage although in the 65°C to 75°C range permanent damage can be avoided by limiting the exposure time to the temperature to very short "flash" exposures. It is preferred that the temperature of the endothelium does not exceed 55°C during the application of the light energy. The invention optionally provides for a transparent heat sink to prevent damage to the anterior surface of the corneal tissue while exposing the stromal collagen portion of corneal tissue to the light energy. The stromal collagen is heated to a temperature of about 55°C to about 75°C. The invention utilizes a coupling device during the application of light energy to couple light energy onto the cornea with precise control of position and initial temperature while providing protective functions for corneal structures that are not being reshaped. The coupling device can also provide a mold or template for the predetermined anterior corneal curvature.

For purposes of this invention it is important to understand the interrelationship of light energy, stromal temperature, heat energy, heat sink, physiological rearrangement of stromal collagen, and time. Functionally, the purpose of this invention is to cause a predetermined, beneficial rearrangement of the anterior stromal collagen without initiating a wound healing response. In order to accomplish the desired rearrangement a specific quantum of light energy is applied to the anterior stroma of the cornea. It is intended that enough light energy be applied to cause the desired physiological change to the anterior stroma without at the same time initiating a wound-healing response in the cornea and without damaging the heat sensitive endothelium, epithelium or Bowman's membrane. Experimentation has verified that a safe temperature range to accomplish the intended rearrangement is 55°C to 65°C. However, stromal temperatures up to 75°C can be tolerated, and will result in the desired physiological rearrangement, if the exposure to temperatures from 65°C to 75°C is maintained at "flash" duration. If the stromal temperature is maintained at 65°C - 75°C for an extended time period, e.g., one second or more, then although the desired physiological rearrangement will occur, the undesirable wound-healing response will also be initiated.

It is likewise important for an understanding of the physiological effect of this invention to understand the desired depth of penetration of the changes in the cornea. As has been previously stated absorption coefficients of 30 cm⁻¹ to 100 cm⁻¹ and 100 cm⁻¹ to 300 cm⁻¹ are preferred in the practice of this invention. This corresponds to a cornea depth of 33-333 microns which would indicate that a physiological change was taking place outside of the stroma at the smallest penetration depths (e.g. 33-60 microns). However, the use of a heat sink on the exterior surface of the cornea permits cooling of the epithelium and Bowman's layer so that heat transfer modifies the depth of penetration of physiological changes. It should be understood that "peak" temperatures would occur only in the stroma with lesser temperature changes occurring outside the stroma being insufficient to cause either a wound healing response or physiological change.

It is the purpose of the current invention to provide a noninvasive method for performing accurate, controlled reshaping of the anterior surface of the cornea in order to correct ocular refractive errors. The invention uses a light source emitting a wavelength or wavelengths with correct optical penetration depths to induce thermal changes in the corneal, stromal collagen without damaging the viability of the corneal endothelium or the anterior surface of the corneal tissue and without causing a sufficient wound-healing response to lead to long-term or permanent corneal reshaping. The light source is coupled with light delivery and control means for producing the required radiant exposure time and geometric pattern in order to achieve the desired change in the shape of the cornea (sometimes referred to as a physiological rearrangement of the stromal collagen). Anterior corneal surface cooling by a transparent heat sink may be used to prevent damage to the endothelium and to Bowman's layer and to insure desired temperature at preferred depths of the stroma. Although the method is described herein as being performed only one time, repeated applications of the method may be desirable or necessary and are included within the scope of the invention.

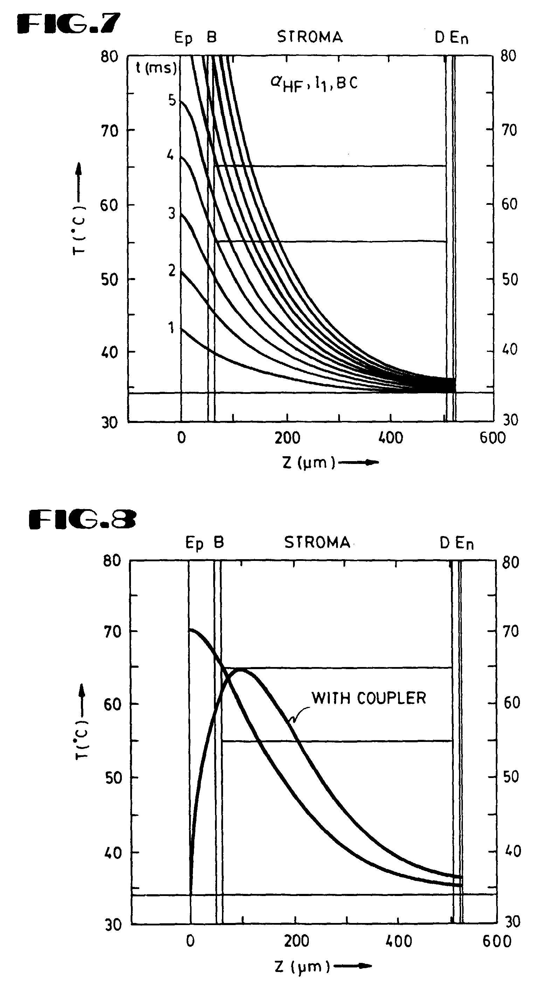

The curvature of the cornea in part controls the refraction of the light received by the lens of the eye. Ocular refractive errors of the eye can be corrected by changing the shape of the cornea. Figure 3 identifies the various portions of the human eye schematically. As is apparent from the representation of the human eye in Figure 3, only a small portion of the cornea is used for actual vision. This portion of the cornea is typically referred to as the central optical zone or central visual zone. The typical central optical zone is 4 millimeters in diameter. Referring now to Figure 4, a cross-section of the cornea is shown together with its various layers. As represented schematically, the epithelium is several layers thick and approximately 40 microns in depth, Bowman's membrane is thinner, approximately 15 microns thick, and the stroma layer is the thickest structure, typically about 450 microns thick. For purposes of this application the anterior portion of the stroma is the anterior one third of the stroma or approximately the first 150 microns of the stromal tissue.

The stroma is composed of various internal layers with the arrangement of the layers being the least organized in the anterior section of the stroma. For purposes of this application, corneal tissue shall refer to the entire corneal structure as shown in Figures 3 and 4. The anterior portion of the stroma (as opposed to cornea) is that portion that demonstrates a more random lamellar structure and typically corresponds to the anterior-most 150 microns. The organic matter within the corneal stroma is primarily made up of Type I collagen. The Type I collagen of the cornea, before heat treatment, is composed of triple helix strands of polypeptides. The polypeptides are held together by hydrogen bonds between the polypeptide strands. On heating the collagen reforms so that the overall length is decreased. This thermal modification phenomenon is called collagen shrinkage. One object of this invention is to allow for ocular changes in the stroma without harming the viability of the other layers. Although some damage is inherent in this method, retaining viability means that the eye continues to function optically and that the cellular layers continue to live and regenerate.

Heating the collagen of the stroma to a temperature of at least 55° to 58°C and up to a maximum of about 75°C causes the collagen to shrink thereby changing the shape of the cornea. This heating process can be effectuated by directing light energy onto the cornea to cause absorption of energy to heat the stromal collagen to the desired temperature. This is done by providing a light source that radiates light energy that is characteristically deposited within a specified depth of the corneal tissue. Light energy as used in this application is not limited to the visible spectrum. The selection and control of the source of light energy that induces the thermal changes to the cornea is critical. The variables used to select the appropriate amount and type of light energy are wavelength, irradiance level, and time (duration). It is essential that these three variables be selected so that the amount of light is functionally effective to produce a predetermined change in the anterior portion only of the stroma. The light source can be either a laser (coherent light) or a non-laser (non-coherent light) light source providing it emits radiation of the appropriate wavelengths to be absorbed within the anterior portion of the stroma without penetrating deeply into the eye in a manner that can damage the endothelium of the cornea or other structures of the eye. The light radiation must also be of a type that can be directed onto the cornea and controlled to produce the appropriate thermal changes.

It should be understood that the term wavelength includes wavelengths of slightly greater and slightly smaller size and is often described for purposes of this application as "one or more wavelengths." It has been found in the current invention that the optimum wavelength ranges are about 1.87 microns to about 2.08 microns and about 2.55 microns to about 2.7 microns. Light within this range of wavelengths is absorbed primarily in the anterior portion of the stroma (See Fig. 5). In general, this invention includes light having wavelengths that are absorbed within a penetration depth of 33 to 125 microns within the cornea. Since human corneas are typically 500 microns or more in thickness the initial absorption of light energy at these wavelengths does not heat the corneal endothelium significantly thus preventing damage to this vulnerable structure. By controlling the duration and irradiance level of light emitted at these wavelengths, substantial thermal conduction of the absorbed light energy can be prevented so that the conducted heat does not damage the corneal endothelium.

The selection and control of the source of light energy that induces the thermal changes to the cornea is critical. The variables used to select the appropriate amount and type of light energy are wavelength, irradiance level, and time (duration). It is essential that these three variables be selected so that the amount of light is functionally effective to produce a predetermined change in the anterior portion only of the stroma without at the same time causing a wound healing response.

Light sources should be used which produce wavelengths for which the absorption coefficient of corneal tissue is in the range of 30 cm⁻¹ - 100 cm⁻¹ (wavelengths of 1.87 - 2.08 microns) and 100 cm⁻¹ - 300 cm⁻¹ (wavelengths of 2.55 - 2.7 microns), corresponding to optical penetration depths in the range 33.3 - 333 microns. Light sources producing absorption coefficients between 30 cm⁻¹ - 100 cm⁻¹ or 100 cm⁻¹ - 300 cm⁻¹ have wavelengths in the range of 1.87 - 2.08 µm or 2.55-2.70 µm, respectively, for the ordinary physiological temperatures of the cornea. At elevated temperatures, however, there may be changes in absorption coefficients and resulting changes in depth of penetration of the cornea. Therefore, it should be understood that the absorption coefficients and related penetration depths are for temperatures at or near physiologically typical temperatures.

One preferred light source is a hydrogen fluoride light source. The most preferred light sources are holmium, erbium, thulium and uranium based non-coherent light sources, or combinations thereof. The light source is tuned to produce only those wavelengths of radiation that are characteristically absorbed in the first 33 to 125 microns of the cornea. The wavelengths chosen for use in this invention fall within the range of approximately 1.87 - 2.08 microns and 2.55 - 2.7 microns. An example of one light source that can be utilized in the current invention is a modified Helios hydrogen fluoride mini-laser (Helios Inc., Longmont, CO). This modified laser system uses special resonator optics that are designed to allow laser action on certain hydrogen fluoride wavelengths while suppressing all other wavelengths.

To achieve treatment of only the anterior portion of the stromal collagen, either an incoherent light source or laser light source, either with a wavelength of 1.87 - 2.08 µm or 2.55-2.70 µm, can be used. Two possible materials to form lasers in the acceptable wavelength range are listed as follows:
(1) KCl:Li F_{A}(II) color center crystals can be used to fabricate tunable color center lasers in the wavelength range 2.5-2.9 µm. At a low temperature, these lasers have very high fluorescence quantum yields of conversion from visible light to infrared light. Conversion from visible light to infrared light is required so that temperature of the stroma is increased. Fig. 5 shows the measured fluorescence and relative laser efficiency taken from L. F. Mollenauer, "Broadly Tunable Lasers Using Color Centers", in S. Haroche, et al., Laser Spectroscopy (Springer-Verlag, New York, 1975) pp. 227-238. The solid curve of Fig. 5 is the luminescence and dots show the 1/P threshold.
(2) Erbium-doped crystals, such as Er³⁺:YAG or trivalent erbium ions doped in yttrium aluminum garnet, are used to make fixed frequency solid state lasers in the wavelength range 2.7-3.0 µm. These lasers also have high fluorescence quantum yields of conversion from visible light to infrared light. Another dopant such as chromium ions in combinations such as Cr:Er:YAG and Cr:Er:YSGG permits broadband pumping to achieve additional power from erbium ions by energy transfer from chromium ions. Besides the above two listed light source materials, other materials may be used to form efficient light sources in the wavelength region 2.55-2.7 µm.

Figure 6 is a graphic representation of temperature in the various layers of the cornea during the application of light energy using various laser light sources at a constant time and irradiance level. Note that the HF laser (at a predetermined and selected wavelength of approximately 2.61 µm for the calculation shown) heats the anterior portion of the stroma to the correct temperature while keeping the endothelium cool and the epithelium at a high, but moderate, temperature. Temperatures at the surface of the cornea are in a range easily controlled by a heat sink. Under the same conditions, the CO₂ laser generates very high temperatures within the epithelium and within Bowman's membrane leading to extensive thermal damage to these structures. The temperature then very quickly drops below the effective temperature of 55°C because penetration into the cornea is so poor. The primary mechanism of energy deposition is thermal conduction of heat into the tissue interior from the epithelium where it is absorbed. Again under the same conditions, the Ho:YAG laser does not generate sufficient heating of the anterior portion of the stroma to produce collagen shrinkage and corneal reshaping; the Ho:YAG laser does, however, heat the endothelium to nearly the same temperature as that obtained in the anterior portion of the stroma since the wavelength (2.1 µm) of the Ho:YAG laser is much more readily transmitted through corneal tissue.

The light source is combined with a means of directing and controlling the light beam from the light source accurately onto corneal surfaces. This will generally include an optical delivery system consisting of beam directing or focusing optics together with an optical shutter or related materials and devices. The optical delivery system allows control of the delivery parameters such as geometry and dose to produce the desired predetermined reshaping of the cornea. It also allows the control of the irradiance level and duration of the light energy directed upon the eye necessary to heat the collagen in the cornea to the point where it will shrink but not be destroyed and to prevent conduction of the thermal energy that could damage the endothelium.

The preferred duration of exposure of the cornea to or time for application of the light energy is less than about 1 second. Most preferably the time exposure is from about 10 milliseconds to about 100 milliseconds, with the most preferred duration being approximately 10 to 50 milliseconds. The light energy is actually applied in an intermittent or pulse form with each pulse being less than 1 second. The level of irradiance is typically selected to be a level wherein absorption is substantially linear. In the most preferred embodiments of this invention, the irradiance level is less than 1 x 10⁸ W/cm² and preferably less than 1 x 10³ W/cm² and most preferably wherein the absorption coefficient is within ranges of about 30 cm⁻¹ -100 cm⁻¹ or 100 cm⁻¹ to 300 cm⁻¹. The variables of wavelength, duration and irradiance are highly interdependent. These variables must be interrelated in a way that a functional amount of light is delivered to the cornea to make the desired predetermined physical changes in the curvature of the cornea without eliciting a wound healing response. A preferred interrelationship of variables includes wavelengths of 1.87 to 2.08 microns or 2.55 to 2.7 microns, a duration of less than 1 second and an irradiance level wherein the absorption coefficient is 30 cm⁻¹ - 100 cm⁻¹ or 100 cm⁻¹ to 300 cm⁻¹. Figure 7 shows graphically the effect of exposure time at a fixed wavelength and irradiance level.

Various geometric patterns and temporal periods of radiation produce corrections of different types and magnitude of ocular refractive errors. Referring now to Figure 9, various geometric patterns and spatial orientations of treatment zones are shown that provide the desired corrective effect. Tangential lines, radial lines, annular rings, and combinations have been shown to be useful in obtaining corrective measures with this invention. In all cases the method results in patterns of shrinkage.

It is critical that at no time during or after application of the functionally effective dose of light in association with the method of this invention is a substantial wound-healing response initiated in the cornea and specifically, in the stromal tissue of the cornea. In this connection, a substantial wound-healing response is one that causes stromal collagen synthesis and regrowth that produces a change in curvature of the anterior surface of the cornea. The corneal wound-healing response in man is complex and not perfectly understood [R.W. Beuerman, C.E. Crosson, and H.E. Kaufman (editors), Healing Processes in the Cornea (Gulf Publishing Co., Houston, TX, 1989)].

Following a substantial wound to the stromal tissue, a sequence of processes occurs that result in the synthesis of new collagen that is initially present in the form of disorganized fibers of nonuniform diameters and irregular orientations. These new collagen fibers degrade corneal transparency since they scatter light. Over a period of several months, the new collagen fibers may be transformed, at least in part, into new stromal lamellae that have more organized structures that transmit light properly without scattering. As these new stromal lamellae are formed, the dimensions and mechanical properties of the stromal tissue change, thereby causing changes in anterior corneal shape. In the present invention, a substantial wound-healing response is avoided by careful control of the nature and extent of stromal collagen alternation. Therefore, the results of the corneal reshaping produced by application of a functionally effective dose of light are predictable and controllable and are not subject to long-term modification due to a substantial wound-healing response. Alternate procedures such as excimer laser photorefractive keratectomy suffer from instability of refractive correction due to long-term stromal regrowth and corneal reshaping.

In one embodiment of the invention the output beam of radiation from a hydrogen fluoride chemical laser is directed by a series of beam steering mirrors onto an X-Y scanner. The scanner is driven by computer controlled electronics to produce a swept beam that is directed onto a focusing lens that produces a specified beam diameter on the front surface of the cornea. The focused beam is applied over the front surface of the cornea with a specified application rate and geometric pattern. A computer controlled optical shutter is used to control the temporal duration of application of the slewed beam which may be continuous or interrupted during laser irradiation of the cornea. Many variations and combinations of optical delivery systems are available and are known to those skilled in the art. Any optical delivery system which will provide the precision necessary to practice the current method will suffice.

A means to measure the curvature of the cornea before, during and after the cornea reshaping process is utilized. Any keratometric device, such as a video keratometer, can be used to produce accurate corneal topographic maps of the cornea. The corneal topographic maps, together with refractive measurements of the eye, are used to establish the geometry and dose of light energy required to produce correction of ocular refractive errors. After the application of light energy, keratometric measurements are performed to produce corneal topographic maps that verify that the desired correction has been obtained. Keratometric measurements may also be made during the cornea reshaping process as multiple applications of light energy may be needed to reach the correct "end point" (an emmetropic eye). Examples of keratometers which may be used include the EyeSys Corneal Analysis system, and the PKS-1000 photokeratoscope (Sun Contact Lens Co. Ltd., Palo Alto, CA). A preferred keratometer will provide digitized output from which a visual display is producible to show the cross-sectional profile of the anterior surface curvature of the cornea.

Also provided by this invention is a means for cooling the anterior surface (understood to be the epithelium and Bowman's membrane) of the cornea during the reshaping process to prevent damage to the corneal epithelium and to Bowman's membrane. This is done by providing a transparent heat sink. It will be obvious to those skilled in the art that the heat sink must be effective enough to prevent damage to the epithelium and Bowman's membrane but not so efficient as to prevent the necessary amount of collagen shrinkage to provide the desired ocular refractive correction. Heat sinks that are suitable for anterior surface cooling include transparent fluids, either gas or liquid, which can be circulated over the anterior corneal surface to provide cooling by forced convection. Any transparent fluid may be used which will not harm the eye and which will serve to adequately cool the cornea. A transparent solid heat sink, such as a hard contact lens which has sufficient thermal conductivity, thermal diffusivity, and heat capacity to provide cooling by conduction may also be used.

As shown in Figure 9, it is important that the light energy be applied in a spatially localized pattern. The localized pattern includes any number of radial lines, transverse lines, a pattern of spots or annular rings depending upon the desired type and degree of correction. To assist in such correction, a specifically designed coupler, often utilized as a heat sink, may also be used. Such a coupler can be made of any of a number of materials but most preferably of Infrasil quartz or sapphire. With such materials, a coupler can be created which cools the anterior surfaces of the cornea to prevent inappropriate heating and damage. The coupler can also be used as a mask to prevent accidental exposure of the non-treated portions of the cornea to any light energy and as a restraint to prevent movement of the eye. Additionally, thermocouples can be attached to the coupler to monitor temperatures and the coupler can be mounted in a thermostatically controlled chamber to provide reproducible pre-operative corneal temperature. Finally the coupler can be used to accurately position the pattern of localized light application.

In one embodiment of the current invention, the transparent solid heat sink or coupler is designed and constructed with the correct curvature in contact with the anterior surface of the cornea to provide a template or mold for the control of the cornea reshaping process. In this embodiment the correct "end point" for cornea reshaping is produced by simultaneously shrinking, swelling, and molding the corneal stromal collagen into the correct final shape. Stromal collagen is a "thermoplastic" material that can be thermally formed into new shapes when it is subjected to mechanical forces. During thermal processing at the collagen shrinkage temperature, collagen shrinks along the main fiber axis direction to a new length that is determined in part by the tension acting along the fiber direction [J.C. Allain et al., *Connective Tissue Research*, 7, 127-133 (1980)]. Collagen swells transverse to the main fiber axis direction.

At elevated temperature (i.e., at the corneal shrinkage temperature), collagen is much less rigid due to the loss of weak bonds (termed hydrogen bonds) that hold collagen molecules in a precise structural pattern (a triple helix) at physiological temperature. Hence, at the collagen shrinkage temperature, stromal collagen is much more plastic and is capable of being molded into a new shape. Once formed into a new shape at the collagen shrinkage temperature, the stromal collagen may then be cooled to physiological temperature. At physiological temperature, new hydrogen bonds form and these tend to preserve the stromal collagen in its new shape. Sapphire and Infrasil quartz (a type of quartz that has high transparency to the preferred wavelengths of functionally effective light) are examples of appropriate solid materials that are transparent to the wavelengths of functionally effective light, that have good thermal properties to serve as a heat sink, and that have mechanical rigidity to act as a template or mold for the reshaping of the anterior surface of the cornea.

Figure 8 is a graphic representation of temperature in the various layers of the cornea using a hydrogen fluoride laser. The graph shows the effectiveness of using a heat sink or coupler. With the extra control provided by the coupler, light energy of a higher irradiance level with a longer exposure time resulted in harmless temperatures in the epithelium and Bowman's membrane while allowing functionally effective temperatures within the anterior portion of the stroma.

A typical laser apparatus for practicing this invention is described below. The light source, a hydrogen fluoride chemical laser or erbium-based laser, Is installed either in the patient treatment room or in a remote location. The light source beam either is propagated through an optical path in air or is coupled into a fiber optic cable. The propagated or coupled beam is then directed onto beam steering, scanning, and focussing optics. An optical shutter is also incorporated within the beam train to provide the correct exposure duration. The beam delivery system includes the scanning system that controls the movement of the laser beam on the predetermined and preselected portion of the patient's cornea.

Additionally, the shutter and scanning system are computer controlled to synchronize their actions and to obtain accurate delivery of the functionally effective light beam onto the patient's cornea. The final delivery optics of the optical delivery system are mounted securely on the patient treatment table. These optics have XYZ coordinate translation adjustments and ΘΦ angular adjustments to permit the beam to be aligned and positioned accurately with respect to the eye that is to be treated.

A beamsplitter is used to sample a small portion (typically, a few percent) of the final output beam that is to be directed onto the patient's eye. This small portion of the beam is directed to beam diagnostic instrumentation to measure laser beam parameters such as power, spot size, and irradiance distribution. The patient sits upright at a slit lamp that includes a chin rest, a head mount for accurate positioning, and an accessory mount that contains the coupler to provide protection to the anterior surface of the cornea, shape the cornea by action of a template surface, mask the surface regions of the cornea that are not to be treated, and thermostatically control the initial corneal temperature. The patient is looking horizontally forward into the coupler and a fixation light source. The treatment light beam is directed horizontally forward through the coupler onto the patient's cornea that is to be treated.

The physician who performs the treatment uses a slit lamp microscope, together with a visible tracer light beam (from a low energy visible light source) that is collinear with the treatment beam, in order to verify the proper positioning of the treatment beam.

One embodiment of the apparatus of this invention is illustrated schematically by the flow chart in Figure 2 while a more preferred embodiment is shown in Figure 2A. This flow chart is intended to show a control means utilized by the physician as the focal point for controlling all variables and light emission devices. The control means is connected to the laser system, the beam delivery system, the scanning system and the keratometer. A light source (in the figure, a laser system) is used to produce functionally effective light. The beam of light is delivered and scanned to produce the correct pattern and dose of functionally effective light on the anterior surface of the cornea. A coupling means is used to provide a heat sink for protection of the anterior surface of the cornea, a template for reshaping the anterior surface of the cornea, a thermostat for accurate and reproducible temperature control prior to laser treatment, a mask for protection of regions of the anterior surface of the cornea that are not intended to be treated, and a positioner/restrainer for accurate positioning of the light source beam on the anterior surface of the cornea and for restriction of eye movement during the treatment. A keratometer is used to perform corneal topography measurements before, during, and after the procedure.

A control means is used to provide predetermined values of pattern and dose of the laser beam on the anterior surface of the cornea. It is suggested that the control means have all the controls necessary for the surgeon to have complete control of the operation including suitable displays of the operation variables showing what has been preselected and what is actually delivered. For example, the pattern of the laser beam should be preselected and be capable of being varied by the surgeon. The pulse duration, the number of pulses to be delivered, the number of pulses actually delivered to a particular location on the eye and the power of each pulse should also be controlled. The control means may be a suitable computer with a terminal at the surgeon's location allowing display of all elements of the operation which may be of interest to the surgeon.

A typical non-coherent light energy source and apparatus for practicing the method of this invention is shown in Figure 2A. The essential components of the system are labelled and identified in Figure 2A. The light source, a fluorescer that is activated by a diode laser, is installed in a patient treatment room. Activation light from a diode laser is coupled into optical fibers that are mounted in a fiber optic array. Each proximal tip of each optical fiber is positioned separately by a translation stage driven by a computer-controlled position controller so that the diode laser beam is properly focused into each proximal tip. The diode laser is driven by a pulsed power supply that is also computer-controlled. With this system, a controlled and pre-specified amount of diode laser light is directed into each optical fiber for a pre-determined time.

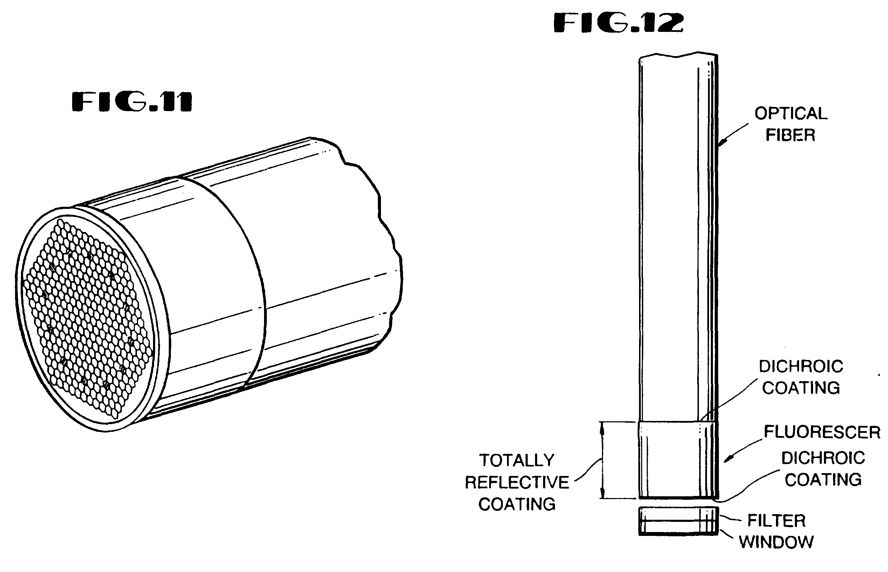

The fiber optic array guides diode laser light pulses to the array of fluorescer elements schematically shown in Figure 11. In this figure each dark element has been activated to form an annulus of fluorescers that are located at the distal ends of optical fibers through which diode laser light has been channeled. With this system, a predetermined pattern of individual fluorescer elements is activated in a predetermined sequence for predetermined periods of time.

Figure 12 shows a detail of one optical fiber/fluorescer assembly. The optical fiber is bonded to the fluorescer and efficiently transmits diode laser light near a wavelength of 785 nanometers into the fluorescer. Fluorescence light of functionally effective wavelengths radiates from the fluorescer, through an optical filter (to restrict fluorescence to a predetermined wavelength band and to remove any remaining diode laser light), and through a window/coupler into the corneal tissue to be treated. A dichroic optical coating is used at the optical fiber/fluorescer interface to transmit diode laser light and to reflect fluorescer light. The fluorescer has a totally reflective optical coating on its sides and also has a dichroic optical coating on its exit face to transmit fluorescer light while reflecting diode laser light. All of these optical coatings improve the efficiency of projecting fluorescer light into the corneal tissue to be treated.

It is essential to understand that one of the primary advantages enjoyed by the practice of this invention is the noninvasive nature of the application process and the fact that no substantial wound-healing response is produced in the cornea. Although the changes to the corneal shape and curvature are long-term physical changes, there are controls in place that prevent the likelihood of there being any risk whatsoever to the patient. It is important to note that the viability of the corneal endothelium, a delicate and critical layer to human eye sight, together with other essential visual components of the eye are maintained throughout the procedure.

### EXAMPLE

The following example of an animal procedure on a New Zealand white rabbit was performed to demonstrate the method required to correct myopia by flattening the central anterior surface of the cornea in an axisymmetric pattern with its origin on the central visual axis. Other procedures have also been performed to demonstrate the methods required to correct hyperopia by steepening the central anterior surface of the cornea in a like axisymmetric pattern and to correct astigmatism by unsymmetric treatment so as to produce different values of corneal flattening and/or steepening along different meridians of the cornea.

Following administration of a general anaesthetic, the rabbit was placed on a small table that included a head mount and positioner for accurate alignment of diagnostic instruments and for accurate delivery of a hydrogen fluoride chemical laser beam (from a modified Helios Inc. chemical laser system equipped with a diffraction grating for tuning output wavelength) onto the rabbit cornea. The table is mobile, permitting it to be moved from the laser treatment area to each of several diagnostic instrument stations while the rabbit head and eye are kept in the proper orientation for either laser delivery or diagnostic measurements.

Prior to laser treatment, several pre-treatment measurements were made on both eyes of the rabbit. First the corneal thicknesses were determined using a DGH Technology, Inc. Model DGH500 ultrasonic pachymeter. Next, the corneas of both eyes were viewed through a Zeiss slit lamp microscope to determine that the corneas were smooth and free of surface defects. Then, just prior to laser treatment, the curvatures of the anterior corneal surfaces were determined with an International Diagnostic Instruments, Ltd. Corneascope. Several post-laser treatment measurements of corneal curvature were made using either the Corneascope or an EyeSys Corneal Analysis System. The former instrument produces a Polaroid film record that must be analyzed manually at a later time while the latter instrument utilizes a video camera and computer analysis system to yield near-realtime corneal topography maps. Both instruments provide sufficiently precise corneal curvature measurements to determine the effectiveness of the corneal reshaping procedure.

For laser treatment, the rabbit was positioned so that the eye to be treated was the correct distance from a focussing lens to produce a predetermined laser beam spot size on the cornea. The hydrogen fluoride (HF) chemical laser was tuned by a diffraction grating to produce a single laser emission line; in this example, the HF P_{1→0}(3) transition that lases at 2.608 µm wavelength was used. This predetermined wavelength has been verified in separate infrared spectroscopic measurements on human corneas to provide a correct optical penetration depth of ca. 90 µm to treat anterior stromal collagen tissue. Accurate power and irradiance distribution measurements of the laser beam were obtained to permit determination and adjustment of the laser beam irradiance so as to generate the predetermined irradiance for treatment. The laser beam power incident on the rabbit cornea was approximately 1.0 W. The laser beam spot size was approximately 500 µm diameter, leading to an average irradiance of approximately 500 W/cm². This spot was slewed in the annular pattern illustrated in Figure 9. The diameter of the center of the annulus was 4 mm and the annulus was positioned axisymmetrically around the central visual axis. An X-Y scanner (General Scanning Model gs/EYE optical scanner) was used to slew the beam at a predetermined slew rate so that the complete annular pattern was generated in a predetermined time of 200 milliseconds (ms). An optical shutter was synchronized to permit delivery of only a single annulus on the anterior surface of the cornea. The total delivered energy was 200 millijoules (mJ).

Immediately following treatment, measurements of corneal thickness and corneal topography were made. In addition, slit lamp photomicroscopy was performed to observe corneal changes. Similar measurements of corneal topography of both the treated and untreated (control) eyes were made at intervals following treatment over a period of 24 weeks. Figure 10 shows the measured pre-laser and post-laser values of corneal refractive power for both the rabbit eyes. Note that the refractive (dioptric) power of rabbit eyes normally varies with time due to growth. Therefore, the refractive power of the control eye decreased approximately five diopters over the period of observation. This type of "baseline drift" is present in all non-adult rabbit eyes, including both the treated and untreated eyes of the example rabbit.

The change in corneal shape due to laser treatment is readily apparent in Figure 10. The immediate post-laser treatment value of refractive power for the treated eye is approximately five diopters less than the pre-laser value, so the cornea has been flattened appreciably. At 4 weeks after treatment, Figure 10 shows that some of the initial flattening has been reduced, but the treated eye has nearly three diopters lower refractive power than the control eye. The difference of three diopters central flattening persists at 10 weeks and at 24 weeks following treatment. This persistence of the central flattening is evidence that little or-no wound-healing response that generates stromal regrowth and anterior corneal shape change has occurred.

Examination of slit lamp photomicrographs revealed that the epithelial layer of the treated cornea was damaged initially. Superficial whitening of the treated annulus was noted. This whitening cleared within a few days, leaving little or no "haziness" of the anterior surface. There have been no other complications of the procedure such as the recurrent erosions that have been observed by other workers who performed thermokeratoplasty on both animal and human patients.

Improved experiments have been carried out on *in vitro* eyes using a heat sink material to protect anterior corneal structures from thermal damage. Identical laser treatments have been applied to the same eye with, and without, a heat sink coupler in place. Predetermined functionally effective doses of light have been delivered using the hydrogen fluoride chemical laser source to demonstrate the effectiveness of the heat sink coupler in preventing damage to the corneal epithelium and the Bowman's layer while causing corneal reshaping to generate correction of myopia.

## Claims

1. Apparatus for non-invasively modifying the shape of a cornea in order to correct ocular refractive errors comprising a source of light energy of a wavelength suitable for absorption by tissue containing collagen tissue in the cornea; means for controlling the radiation source; and means for directing radiation from the source to the tissue
CHARACTERISED IN THAT
the directing means comprises fibre optic means for receiving radiation from the source at first ends thereof and transmitting such radiation for delivery from second ends thereof, and a coupler for coupling radiation onto the face of a cornea is disposed adjacent the second ends of the optical fibres, the second ends thereof being positioned such that radiation spots can be delivered through the coupler to a cornea, means being provided for selectively locating such delivered spots of radiation in a pattern to the cornea, and the locating means and the controlling means being operable to modify the shape of a cornea by selective shrinkage therein of tissue containing collagen.

2. Apparatus according to Claim 1 wherein the coupler provides a mould for the anterior curvature of the cornea.

3. Apparatus according to Claim 1 or Claim 2 wherein the locating means includes means for illuminating the first ends of selected ones of the optical fibres with radiation from the source.

4. Apparatus according to Claim 3 wherein the illuminating means includes means for scanning a beam of said optical radiation among the first ends of selected ones of the optical fibres.

5. Apparatus according to any preceding Claim wherein the source is a source for emitting coherent radiation in the wavelength ranges of 1.87 to 2.08µm or 2.55 to 3µm.

6. Apparatus according to Claim 5 wherein the source comprises a solid state laser.

7. Apparatus according to Claim 5 wherein said source comprises a diode laser.

8. Apparatus according to any preceding Claim wherein the second ends of the optical fibres are positioned to deliver through the coupler to the cornea a specially localised pattern of radiation spots.

9. Apparatus according to any of Claims 1 to 4 including individual elements of fluorescent material positioned at the second ends of the optical fibres to receive optical radiation from the source and direct incoherent fluorescent radiation through the coupler.

10. Apparatus according to Claim 9 including elements positioned between the elements of fluorescent material and their respective optical fibres that reflect the fluorescent radiation and pass the optical radiation from the radiation generating means.

11. Apparatus according to Claim 9 or Claim 10 including a filter positioned over the elements of fluorescent material for passing the fluorescent radiation through the coupler and blocking optical radiation from the source.

## Patentansprüche

1. Vorrichtung zum nicht-invasiven Modifizieren der Gestalt einer Hornhaut, um Brechungsfehler im Auge zu korrigieren, umfassend eine Lichtenergiequelle mit einer Wellenlänge, die sich für die Absorption durch Kollagengewebe enthaltendes Gewebe in der Hornhaut eignet; Mittel zum Regeln der Strahlungsquelle; und Mittel, um Strahlung von der Quelle zum Gewebe zu lenken,
dadurch gekennzeichnet, daß
das Lenkmittel Lichtwellenleitermittel zum Aufnehmen von Strahlung von der Quelle an ihren ersten Enden und Übertragen dieser Strahlung zur Abgabe aus ihren zweiten Enden umfaßt und ein Koppler zum Koppeln von Strahlung an die Oberfläche einer Hornhaut an die zweiten Enden der Lichtwellenleiter angrenzend angeordnet ist, wobei ihre zweiten Enden so angeordnet sind, daß Strahlungsflecke durch den Koppler an eine Hornhaut abgegeben werden können, wobei Mittel vorgesehen sind, um diese abgegebenen Strahlungsflecke in einem Muster auf der Hornhaut zu positionieren, und die Positionierungsmittel und Regelungsmittel so betrieben werden können, daß die Gestalt einer Hornhaut durch selektives Schrumpfen von kollagenhältigem Gewebe darin modifiziert wird.

2. Vorrichtung nach Anspruch 1, worin der Koppler eine Form für die Vorderkrümmung der Hornhaut umfaßt.

3. Vorrichtung nach Anspruch 1 oder 2, worin das Positionierungsmittel Mittel zum Beleuchten der ersten Enden von ausgewählten der Lichtwellenleiter mit Strahlung von der Quelle umfaßt.

4. Vorrichtung nach Anspruch3, worin das Beleuchtungsmittel Mittel zum Abtasten eines Strahls der optischen Strahlung aus den ersten Enden von ausgewählten der Lichtwellenleiter umfaßt.

5. Vorrichtung nach einem der vorangegangenen Ansprüche, worin die Quelle eine Quelle zum Aussenden von kohärenter Strahlung in den Wellenlängenbereichen von 1,87 bis 2,08 µm oder 2,55 bis 3 µm ist.

6. Vorrichtung nach Anspruch 5, worin die Quelle einen Festkörperlaser umfaßt.

7. Vorrichtung nach Anspruch 5, worin die Quelle einen Diodenlaser umfaßt.

8. Vorrichtung nach einem der vorangegangenen Ansprüche, worin die zweiten Enden der Lichtwellenleiter so angeordnet sind, daß sie durch den Koppler an die Hornhaut ein speziell positioniertes Muster von Strahlungsflecken abgeben.

9. Vorrichtung nach einem der Ansprüche 1 bis 4, umfassend einzelne Elemente aus fluoreszierendem Material, die so an den zweiten Enden der Lichtwellenleiter angeordnet sind, daß sie optische Strahlung von der Quelle aufnehmen und inkohärente Fluoreszenzstrahlung durch den Koppler lenken.

10. Vorrichtung nach Anspruch 9, die Elemente umfaßt, die zwischen den Elementen aus fluoreszierendem Material und ihren jeweiligen Lichtwellenleitern angeordnet sind, die die Fluoreszenzstrahlung reflektieren und die optische Strahlung von den Strahlungserzeugungsmitteln durchlassen.

11. Vorrichtung nach Anspruch 9 oder 10, die einen Filter umfaßt, der über den Elementen aus fluoreszierendem Material angeordnet ist, uni die Fluoreszenzstrahlung durch den Koppler hindurchzulassen und optische Strahlung von der Quelle zu blockieren.

## Revendications

1. Appareil destiné à modifier de manière non agressive la forme de la cornée pour corriger les vices de réfraction dans l'oeil, comportant une source d'énergie lumineuse présentant une longueur d'onde appropriée en vue de son absorption par le tissu contenant du collagène dans la cornée ; moyen de contrôle de la source de rayonnement ; et moyen de guidage du rayonnement provenant de la source sur le tissu
**caracterisé en ce que**
le moyen de guidage comporte un moyen à fibres optiques destiné à recevoir le rayonnement provenant de la source à des premières extrémités de celles-ci et à transmettre un tel rayonnement pour fourniture à partir de secondes extrémités de celles-ci ainsi qu'un coupleur, destiné à couper le rayonnement sur la face de la cornée, est placé de manière à être contigu aux secondes extrémités des fibres optiques, les secondes extrémités de celles-ci étant positionnées de telle sorte que les spots de rayonnement puissent être projetés à travers le coupleur sur la cornée, des moyens étant fournis pour pointer sélectivement de tels spots de rayonnement projetés selon un motif sur la cornée, le moyen de pointage et le moyen de contrôle étant règlables afin de modifier la forme de la cornée par un rétrécissement sélectif du tissu contenant du collagène.

2. Appareil selon la revendication 1 dans lequel le coupleur fournit un moule pour la courbure antérieure de la cornée.

3. Appareil selon la revendication 1 ou selon la revendication 2 dans lequel le moyen de pointage comprend un moyen pour illuminer les premières extrémités des fibres optiques sélectionnées à l'aide d'un rayonnement provenant de la source.

4. Appareil selon la revendication 3 dans lequel le moyen d'illumination comprend des moyens de balayage par un faisceau dudit rayonnement optique parmi les premières extrémités des fibres optiques sélectionnées.

5. Appareil selon l'une quelconque des revendications précédentes dans lequel la source est une source émettant un rayonnement cohérent dans les gammes des longueurs d'onde de 1,87 à 2,08 µm ou de 2,55 à 3 µm.

6. Appareil selon la revendication 5 dans lequel la source comprend un laser à semi-conducteur.

7. Appareil selon la revendication 5 dans lequel la source comprend un laser à diode.

8. Appareil selon l'une quelconque des revendications précédentes dans lequel les secondes extrémités des fibres optiques sont positionnées pour projeter à travers le coupleur sur la cornée un motif de spots de rayonnement spécialement localisés.

9. Appareil selon l'une quelconque des revendications 1 à 4 comprenant des éléments individuels en un matériau fluorescent positionnés aux secondes extrémités des fibres optiques pour recevoir le rayonnement optique provenant de la source et guider le rayonnement fluorescent incohérent à travers le coupleur.

10. Appareil selon la revendication 9 comprenant des éléments positionnés entre les éléments en matériau fluorescent et leurs fibres optiques respectives qui réfléchissent le rayonnement fluorescent et laissent passer le rayonnement optique provenant du moyen de génération de ce rayonnement.

11. Appareil selon la revendication 9 ou selon la revendication 10 comprenant un filtre positionné sur les éléments en matérieau fluorescent destiné à laisser passer le rayonnement fluorescent à travers le coupleur et à bloquer le rayonnement optique provenant de la source.
